# EUROPÄISCHE PATENTSCHRIFT

(11) **EP 0 948 936 B1**
(45) Veröffentlichungstag und Bekanntmachung des Hinweises auf die Patenterteilung: **24.05.2006**
(21) Anmeldenummer: 99106096.3
(22) Anmeldetag: 26.03.1999
(51) Int. Cl.: A61B 17/17

(54) **Zielgerät für einen Verriegelungsnagel**
Guide for use with intramedullary nail
Instrument de visée pour clou intramédullaire

(30) Priorität: 09.04.1998 DE 29806564 U
(43) Veröffentlichungstag der Anmeldung: 13.10.1999
(60) Teilanmeldung: 06004135.7
(73) Patentinhaber: Stryker Trauma GmbH, 24232 Schönkirchen (DE)
(72) Erfinder: Simon, Bernd, Dipl.-Ing., 24106 Kiel (DE)
(74) Vertreter: Graalfs, Edo

(56) Entgegenhaltungen:
- EP-A- 0 556 500
- US-A- 4 913 137
- US-A- 4 920 958
- US-A- 5 281 224
- US-A- 5 354 300

## Beschreibung

Die Erfindung bezieht sich auf ein Zielgerät für einen Verriegelungsnagel nach dem Oberbegriff des Anspruchs 1.

In den Unterschenkel- oder Oberschenkelknochen einführbare Verriegelungsnägel weisen zumeist mehrere Querbohrungen auf, durch die hindurch Knochenschrauben geführt werden, um die Verriegelungsnägel sicher im Knochenkanal zu halten. Insbesondere wird dadurch eine Drehsicherung erhalten. So ist in US 4,920,958 eine u-förmige Kontaktfeder mit Führungslöchern für einen anzusetzenden Knochenbohrer vorgesehen, die am proximalen Ende des Marknagels angeschraubt ist und in die in einem bestimmten Abstand, analog zum Marknagel, Führungslöcher eingebracht sind.

Ein besonderes Problem bei Verriegelungsnägeln ist das Auffinden der Querbohrungen beim implantierten Nagel. Hierfür werden daher Zielgeräte verwendet. Eine Kategorie von Zielgeräten arbeitet mit Röntgenstrahlen. Die Querbohrungen des Verriegelungsnagels im Knochen werden auf einem Monitor abgebildet. Ferner findet eine Abbildung eines Zielelements statt. Auf diese Weise ist es möglich, an der Außenseite die Stelle zu markieren, die auf der Achse der Querbohrungen liegt.

Bei einer anderen Kategorie werden Zielgeräte fest mit einem Ende eines Nagels verbunden. Ein bügelartiger Abschnitt weist mindestens eine Durchbohrung auf, deren Achse mit der Achse einer Querbohrung des Nagels ausgerichtet ist, wenn er am Zielgerät angebracht ist. Zur Führung des Bohrwerkzeugs bzw. der Knochenschraube ist auch bekannt, durch die Querbohrung des Zielgerätes eine Führungshülse zu stecken, die bis gegen die Außenseite des Knochens vorgeschoben wird.

Die Führungshülse sitzt zwar passend in der Querbohrung des Zielgerätes, muß jedoch von Hand relativ leicht bewegt werden können, um dem Chirurgen die Arbeit nicht zu erschweren. Dadurch besteht jedoch Gefahr, daß bei der Handhabung des Zielgerätes bzw. beim Bohren und auch beim Einschrauben von Knochenschrauben die Führungshülse nach hinten verrutscht.

Der Erfindung liegt daher die Aufgabe zugrunde, ein Zielgerät für einen Verriegelungsnagel zu schaffen, bei dem die Hülse auf einfache Weise verstellt und in der jeweiligen Position fixiert werden kann.

Diese Aufgabe wird durch die Merkmale des Anspruchs 1 gelöst.

Bei dem erfindungsgemäßen Zielgerät weist der Aufnahmeabschnitt zwei Teile auf, wobei der eine, der einem aufgenommenen Nagel zugekehrt ist, starr mit den übrigen Abschnitten des Zielgeräts verbunden ist, während der zweite Teil gegen Federkraft relativ zum ersten Teil bewegbar ist. Die Querbohrung erstreckt sich durch beide Teile, wobei die Teilbohrungen so angeordnet sind, daß bei entspanntem zweiten Teil die Hülse leicht geklemmt gehalten ist. Wird hingegen der zweite Teil in Richtung des ersten Teils gedrückt, läßt sich die Hülse mehr oder weniger frei in den Teilbohrungen verschieben.

Wird eine Hülse von der Querbohrung des Zielgeräts aufgenommen und in Richtung des aufgenommenen Nagels gedrückt, führt dies zu einer gewissen Verformung des zweiten Teils in Richtung des ersten Teils, wodurch die Hülse gegen einen gewissen Widerstand verschoben werden kann, ohne daß eine zusätzliche Betätigung notwendig ist. Falls hierzu eine zu große Kraft aufzubringen ist, genügt ein leichtes Drücken auf den zweiten Teil, damit die Hülse verschoben werden kann. Hierbei kann die Hülse auch nach hinten geschoben werden, falls sie entfernt werden soll. Wesentlich ist jedoch, daß die Hülse auch mit relativ großem Kraftaufwand nicht aus der Bohrung herausgezogen werden kann, wenn das zweite Teil nicht in Richtung erstes Teil gedrückt wird. Das verschwenkbare zweite Teil wird bei dem Versuch, die Hülse herauszuziehen, vom ersten Teil fort geschwenkt und erhöht die Klemmwirkung. Diese steigt mit aufgebrachter Kraft, wodurch die Hülse gegen ein Zurückschieben sicher gehalten ist.

Es gibt verschiedene Möglichkeiten, die erfindungsgemäße Konstruktion zu verwirklichen. Eine Ausgestaltung sieht hierzu vor, daß erstes und zweites Teil einteilig aus elastischem Werkstoff geformt sind. Es ist natürlich auch denkbar, das zweite Teil am ersten anzulenken und zwischen beiden eine Feder wirken zu lassen.

Eine andere Ausgestaltung sieht vor, daß das zweite Teil am freien Ende des ersten Teils angebracht und von diesem durch einen annähernd parallelen Schlitz getrennt ist.

Das gesamte Zielgerät kann einteilig geformt werden, beispielsweise gegossen werden. Es ist jedoch vorteilhafter, den Aufnahmeabschnitt einteilig und das übrige Gerät einteilig zu formen, da es dann leichter ist, für den Aufnahmeabschnitt einen geeigneten, ausreichend federnden Werkstoff zu wählen. Für die übrigen Abschnitte ist hingegen ein relativ fester und ggf. starrer Werkstoff erforderlich, vor allem wenn das Zielgerät gleichzeitig als Einschlaginstrument dient.

Die Erfindung wird nachfolgend anhand von Ausführungsbeispielen näher erläutert.
- Fig. 1: zeigt perspektivisch eine erste Ausführungsform eines Zielgeräts nach der Erfindung.
- Fig. 2: zeigt die Seitenansicht einer zweiten Ausführungsform eines Zielgeräts nach der Erfindung.
- Fig. 3: zeigt einen Schnitt durch das Zielgerät nach Fig. 2.
- Fig. 4: zeigt eine Seitenansicht des Zielgeräts nach Fig. 2 in Richtung Pfeil 4.

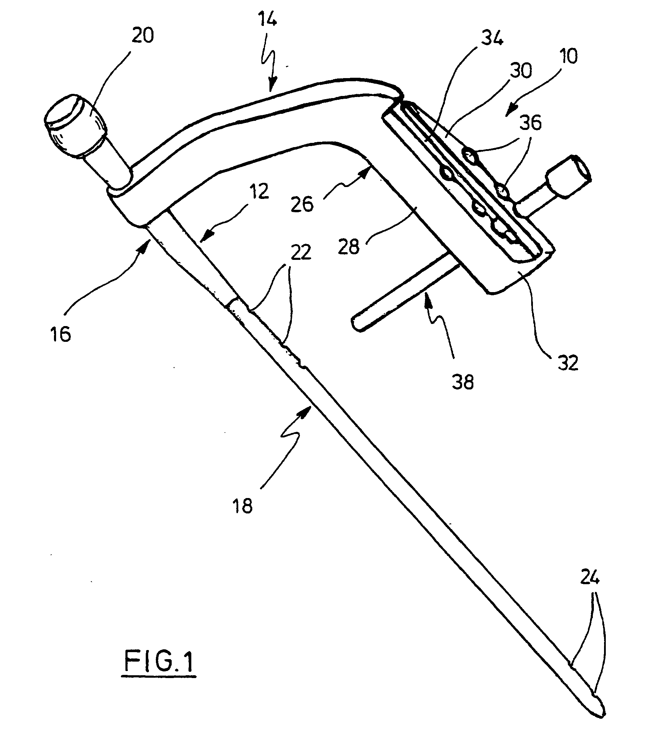

In Fig. 1 ist ein Zielgerät 10 dargestellt, das aus einem ersten Abschnitt 12 und einem bügelartigen zweiten Abschnitt 14 zusammengesetzt ist. Der bügelartige Abschnitt 14 weist am linken Ende eine Durchbohrung auf zur Aufnahme einer Befestigungshülse 16, welche starr in dieser Bohrung befestigt ist. Die Hülse hat am unteren Ende einen Anschlußabschnitt (nicht gezeigt) zur Verbindung mit einem an sich bekannten Verriegelungsnagel 18. Orientierungsmittel des Aufnahmeabschnitts sorgen dafür, daß der Nagel 18 in vorgegebener Drehlage mit der Hülse 16 verbunden wird. Hierzu dient zum Beispiel ein Schraubenbolzen, dessen Kopf bei 20 zu erkennen ist und dessen Gewinde mit einem Innengewindeabschnitt des Nagels 18 zusammenwirkt. Der Verriegelungsnagel 18 weist im oberen Bereich bei 22 Querbohrungen auf und im unteren Bereich Querbohrungen 24. Sie dienen zur Aufnahme von Knochenschrauben (nicht gezeigt), die quer durch den Knochen und durch die Querbohrungen 22, 24 hindurchgeschraubt werden, wie an sich bekannt.

Der Geräteabschnitt 14 weist einen Aufnahmeabschnitt 26 auf, der aus einem ersten Teil 28 und einem zweiten Teil 30 besteht. Das erste Teil ist einteilig mit dem zweiten Abschnitt 14 geformt und starr mit diesem verbunden. Das zweite Teil 30 ist am freien Ende bei 32 an den Abschnitt 28 angebunden und von diesem durch einen annähernd parallel verlaufenden Einschnitt 34 getrennt. Da der Werkstoff, aus dem der Abschnitt 14 geformt ist, federnd ist, kann das Teil 30 auf das Teil 28 zu und von diesem fort verschwenkt bzw. gebogen werden.

Querbohrungen 36 erstrecken sich durch die Teile 28, 30 und dienen zur Aufnahme einer Führungshülse 38, die durch die unterste Bohrung hindurchgesteckt ist. Die Führungshülse 38 kann jeweils annähernd passend von den Bohrungen 36 in den Teilen 28, 30 aufgenommen werden, wobei jedoch die Lage der Bohrungen in den beiden Teilen 28, 30 zueinander derart ist, daß die Hülse in entspannter Lage des Teils 30 leicht klemmend gehalten ist. Wird das Teil 30 ein wenig in Richtung Teil 28 verformt, läßt sich die Hülse 38 leicht in den Bohrungen 36 verschieben. Wird hingegen das Teil 30 in die entgegengesetzte Richtung verformt, wird die Hülse 38 eingeklemmt und kann nicht mehr bewegt werden.

Die Bohrungen 36, insbesondere im Teil 28, sind in ihrer Achse zu den Querbohrungen 22 ausgerichtet. Wird durch die Hülse 38 ein Bohrwerkzeug geführt, bohrt es den Knochen an einer Stelle an, die auf der gemeinsamen Achse einer Querbohrung des Nagels liegt. Für diesen Vorgang wird die Hülse 38 gegen den Knochen vorgeschoben. Ein Zurückschieben der Hülse 38 wird durch die beschriebene Klemmwirkung verhindert. Soll die Hülse gleichwohl entfernt werden, wird das Teil 30 ein wenig gegen das Teil 28 bewegt.

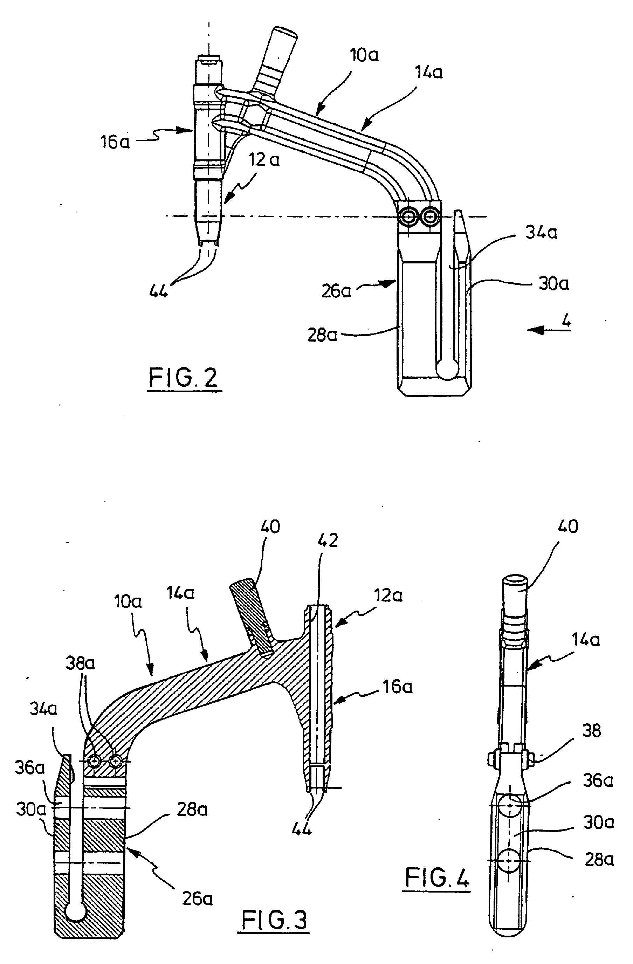

In den Figuren 2 bis 4 ist ein Zielgerät 10a dargestellt, das ähnlich aufgebaut ist wie das nach Fig. 1. Daher werden gleiche Teile mit gleichen Bezugszeichen versehen, denen jedoch ein a hinzugefügt ist.

Man erkennt, daß bei dieser Ausführungsform der Aufnahmeabschnitt 26a über Verbindungshülsen 38a mit dem zweiten Abschnitt 14a verbunden ist. Der Abschnitt 14a ist einteilig mit dem Abschnitt 12a aus einem geeigneten metallischen Werkstoff geformt, während zum Beispiel der Aufnahmeabschnitt 26a aus Kunststoffmaterial besteht. Ansonsten ist der Aufnahmeabschnitt 26a völlig gleich zu dem nach Fig. 1 ausgebildet.

Man erkennt, daß der zweite Abschnitt 14a einen nach oben stehenden Schlagdorn 40 aufweist, der in geeigneter Weise befestigt ist. Man erkennt in Fig. 3, daß der erste Abschnitt 12a eine durchgehende Bohrung 42 aufweist zur Aufnahme eines Befestigungs- bzw. Schraubbolzens zum Anbringen eines Verriegelungsnagels. Zwei Vorsprünge 44 am unteren Ende des Hülsenabschnitts 16a wirken mit nicht gezeigten Ausnehmungen eines Verriegelungsnagels zusammen, um diesem die richtige Orientierung zu geben.

Die lösbare Befestigung des Aufnahmeabschnitts 26a hat den Vorteil, daß verschiedene Aufnahmeabschnitte angebracht werden können je nach eingesetztem Verriegelungsnagel. Muß zum Beispiel im Hinblick auf die Bohrungen 24 des Verriegelungsnagels nach Fig. 1 ein Loch in den Knochen gebohrt werden, bedarf es eines anderen Zielgerätes. Bei der Ausführungsform nach Fig. 2 bis 4 kann daher ein passender Auf nahmeabschnitt 26a an dem übrigen Teil des Zielgerätes angebracht werden.

## Patentansprüche

1. Zielgerät für einen Verriegelungsnagel mit einem ersten Abschnitt, der mit dem zugeordneten Ende des Nagels lösbar verbindbar ist, einem mit dem ersten Abschnitt verbundenen bügelartigen zweiten Abschnitt, der einen Aufnahmeabschnitt aufweist, der annähernd parallel zum Nagel verläuft, wenn der Nagel mit dem ersten Abschnitt verbunden ist und der mindestens eine Querbohrung aufweist für die annähernd passende Aufnahme einer Führungshülse, **dadurch gekennzeichnet, daß** der Aufnahmeabschnitt (26, 26a) aus einem starr mit dem zweiten Abschnitt (14a) geformten, dem ersten Abschnitt (16a) zugewandten ersten Teil (28, 28a) und einen zweiten relativ zum ersten Teil (28a) gegen Federkraft bewegbaren äußeren zweiten Teil (30, 30a) besteht, wobei sich die Querbohrung (36, 36a) durch beide Teile (28, 30, 28a, 30a) hindurcherstreckt und die Bohrung (36, 36a) in den Teilen (28, 30, 28a, 30a) so gewählt ist, daß bei entspanntem zweiten Teil (30, 30a) die Hülse (38) leicht klemmend gehalten ist und bei einer gewissen Verstellung des zweiten Teils (30, 30a) auf das erste Teil (28, 28a) zu in der Bohrung (36, 36a) frei verschiebbar ist.

2. Zielgerät nach Anspruch 1, **dadurch gekennzeichnet, daß** erstes und zweites Teil (28, 30, 28a, 30a) einteilig aus einem elastischen Werkstoff geformt sind.

3. Zielgerät nach Anspruch 2, **dadurch gekennzeichnet, daß** das zweite Teil (30, 30a) am freien Ende des ersten Teils (28, 28a) angebracht und von diesem durch einen parallelen Schlitz (34, 34a) getrennt ist.

4. Zielgerät nach einem der Ansprüche 1 bis 3, **dadurch gekennzeichnet, daß** der Aufnahmeabschnitt (26, 26a) einteilig geformt ist.

5. Zielgerät nach einem der Ansprüche 1 bis 4, **dadurch gekennzeichnet, daß** erster und zweiter Abschnitt (14, 16) einteilig geformt sind.

6. Zielgerät nach einem der Ansprüche 1 is 4, **dadurch gekennzeichnet, daß** der erste Teil (28a) des Aufnahmeabschnitts (26a) über Verbindungselemente (38a) mit dem zweiten Abschnitt (14a) verbunden ist.

7. Zielgerät nach Anspruch 6, **dadurch gekennzeichnet, daß** erstes und zweites Teil (28a, 30a) aus geeignetem Kunststoffmaterial und erster Abschnitt (16a) und übriger zweiter Abschnitt (14a) aus Metall geformt sind.

## Claims

1. A targeting apparatus for a locking nail with a first section which is releasably connectable to the allocated end of the nail, with a bow-like second section connected to the first section, which comprises a receiving section running approximately parallel to the nail when the nail is connected to the first section and which comprises at least one transverse bore for the approximate fitting accommodation of a guiding sleeve, **characterized in that** the receiving section (26, 26a) consists of a first part (28, 28a) rigidly formed with the second section (14a) and facing the first section (16a), and of an outer second part (30, 30a) movable relative to the first part (28a) against spring force, wherein the transverse bore (36, 36a) extends through both parts (28, 30, 28a, 30a) and the bore (36, 36a) in the parts (28, 30, 28a, 30a) is selected such that with a relaxed second part (30, 30a) the sleeve (38) is held in a slightly clamping manner and with a certain adjustment of the second part (30, 30a) towards the first part (28, 28a) is freely displaceable in the bore.

2. A targeting apparatus according to claim 1, **characterized in that** the first and second part (28, 30, 28a, 30a) are formed as one piece from an elastic material.

3. A targeting apparatus according to claim 2, **characterized in that** the second part (30, 30a) is attached on the free end of the first part (28, 28a) and is separated from this by a parallel slot (34, 34a).

4. A targeting apparatus according to one of the claims 1 to 3, **characterized in that** the receiving section (26, 26a) is formed as one piece.

5. A targeting apparatus according to one of the claims 1 to 4, **characterized in that** the first and second section (14, 16) are formed as one piece.

6. A targeting apparatus according to one of claims 1 to 4, **characterized in that** the first part (28a) of the receiving section (26a) is connected to the second section (14a) via connecting elements (38a).

7. A targeting apparatus according to claim 6, **characterized in that** the first and second part (28a, 30a) are formed from a suitable plastic material and the first section (16a) and the remaining second section (14a) are formed of metal.

## Revendications

1. Instrument de visée pour un clou de verrouillage, comportant une première partie, qui est apte à être assemblée de manière amovible à l'extrémité correspondante du clou, une deuxième partie en forme d'arceau, qui est reliée à la première partie et qui comporte un tronçon de réception, qui est à peu près parallèle au clou, lorsque ce dernier est assemblé à la première partie, et qui comporte au moins une forure transversale destinée à recevoir de manière sensiblement ajustée un manchon de guidage, **caractérisé en ce que** le tronçon de réception (26, 26a) est formé par un premier segment (28, 28a), qui est formé de manière rigide avec la deuxième partie (14a) et est orienté vers la première partie (16a), et un deuxième segment (30, 30a) extérieur, mobile par rapport au premier segment (28a) dans le sens opposé à une élasticité, la forure transversale (36, 36a) passant à travers les deux segments (28, 30, 28a, 30a) et la forure (36, 36a) dans les deux segments (28, 30, 28a, 30a) étant choisie de telle sorte que le manchon (38) est maintenu légèrement serré lorsque le deuxième segment (30, 30a) est détendu, et est librement mobile dans la forure (36, 36a) lorsque le deuxième segment (30, 30a) effectue un mouvement déterminé vers le premier segment (28, 28a).

2. Instrument de visée selon la revendication 1, **caractérisé en ce que** le premier et le deuxième segment (28, 30, 28a, 30a) sont réalisés d'un seul tenant dans un matériau élastique.

3. Instrument de visée selon la revendication 2, **caractérisé en ce que** le deuxième segment (30, 30a) est rapporté sur l'extrémité libre du premier segment (28, 28a) et est séparé de celui-ci par une fente parallèle (34, 34a).

4. Instrument de visée selon l'une quelconque des revendications 1 à 3, **caractérisé en ce que** le tronçon de réception (26, 26a) est formé d'un seul tenant.

5. Instrument de visée selon l'une quelconque des revendications 1 à 4, **caractérisé en ce que** la première et la deuxième partie (14, 16) sont formées d'un seul tenant.

6. Instrument de visée selon l'une quelconque des revendications 1 à 4, **caractérisé en ce que** le premier segment (28a) du tronçon de réception (26a) est assemblé à la deuxième partie (14a) par l'intermédiaire d'éléments d'assemblage (38a).

7. Instrument de visée selon la revendication 6, **caractérisé en ce que** le premier et le deuxième segment (28a, 30a) sont réalisés dans une matière plastique appropriée et la première partie (16a) et le reste de la deuxième partie (14a) sont réalisés en métal.
